# EUROPÄISCHE PATENTANMELDUNG

(11) **EP 2 341 339 A2**
(43) Veröffentlichungstag der Anmeldung: **06.07.2011**
(21) Anmeldenummer: 10197268.5
(22) Anmeldetag: 29.12.2010
(51) Int. Cl.: G01N 33/24, G01N 21/55

(54) **Analyse von Bodenproben**

(30) Priorität: 30.12.2009 AT 20502009
(71) Anmelder: IPUS Mineral- & Umwelttechnologie GmbH, 8786 Rottenmann (AT)
(72) Erfinder: Lesjak, Meinhard, 8943, Aigen im Ennstal (AT)
(74) Vertreter: Burger, Hannes

(57) **Zusammenfassung**

Die Erfindung betrifft ein Verfahren zur Bestimmung von Bodenproben, nach dem eine definierte Menge einer Bodenprobe in einem flüssigkeitsdichten Behältnis mit einer definierten Menge eines flüssigen Elutionsmittels eluiert wird, und danach das Eluat von einem pulverförmigen Zeolithmaterial durch ein Sieb hindurch aufgesaugt wird, und das so gewonnene eluatbeladene Zeolithmaterial getrocknet und mittels Reflexionsspektroskopie im Bereich der Wellenlängen von 400 nm bis 2500 nm analysiert wird, wobei aus dem Reflexionsspektrum die quantitativen Gehalte der wasserlöslichen organischen Substanz, des wasserlöslichen organischen Stickstoffs und Kohlenstoffs, sowie der wasserlöslichen Anteile von Kalium, Natrium, Calcium, Magnesium und Phosphor der Bodenproben durch statistischen Vergleich mit den Reflexionsspektren einer Gruppe von gleich behandelten Bodenproben ähnlicher und bekannter chemischer Zusammensetzung mittels bekannter multivariater Berechnungsmethoden ermittelt wird.

## Beschreibung

Die Erfindung beschreibt ein Verfahren zur Bestimmung von Bodenproben.

Zur Bestimmung der pflanzenwirksamen Nährstoffe von Böden werden üblicherweise chemische Analyseverfahren eingesetzt, die oftmals in Standardmethoden festgeschrieben sind. Diese Analysenverfahren zielen darauf ab, die Quantität oder Konzentration eines bestimmten Stoffes oder einer Stoffgruppe durch ein bestimmtes chemisches oder physikalischchemisches Verfahren zu bestimmen. Zur Ermittlung mehrerer Stoffe in einer Probe werden meist verschiedene Analysenverfahren angewendet, sodass eine große Anzahl verschiedener zu analysierender Stoffe meist mit der Anwendung vieler verschiedener Analyseverfahren verbunden ist. Vereinzelt ist es möglich, Stoffe, die eine bestimmte chemische Ähnlichkeit aufweisen, mit einem einzigen Analyseverfahren zu bestimmen. Dies betrifft beispielsweise die Flüssigkeitschromatographie einer Gruppe von Kationen, zu denen die Kationen der Elemente Kalium, Natrium, Calcium oder Magnesium gehören. Auch für die Analyse einer Gruppe von Anionen wie Karbonat, Chlorid, Sulfat, Phosphat und Nitrat stehen ebenfalls Analyseverfahren mittels Flüssigkeitschromatographie zur Verfügung, in denen diese Stoffe im selben Arbeitsgang bestimmt werden. Durchwegs üblich in der Praxis ist auch die simultane Bestimmung von schweren Elementen der gelösten Stoffe aus einer wässrigen, chemisch aufgeschlossenen Lösung durch Atomabsorptions-spektroskopie oder durch Massenspektroskopie mit induktiv gekoppeltem Plasma (ICPMS).

Jedoch ist bisher keine Methode verfügbar, die im selben Arbeitsgang aus dem Eluat einer Bodenprobe die Analysenparameter wasserlösliche organische Substanz, wasserlöslicheer organisch gebundener Stickstoff, Ammonium-Stickstoff, wasserlösliche Anteile von Kalium, Natrium,Calcium, Magnesium und Phosphor mit einer für die Bestimmung der Bodenfruchtbarkeit genügenden Genauigkeit quantitativ ermitteln kann.

Die wässrige organische Substanz wird oftmals auch in der Abkürzung WOS genannt und stellt einen wichtigen Nährstoffparameter von Böden für die Pflanzenzucht dar. Er wird in der konventionellen chemischen Analytik am einfachsten durch Eindampfen eines Bodeneluates bis zur Trockene bei 105°C und anschliessender Gewichtsmessung des Rückstandes gemessen.

Organisch gebundener Stickstoff in Bodenproben wird üblicherweise nach dem Kjeldahl-Verfahren analysiert. Gemäß der Vorschrift EN 25663 H 11 werden zur Ermittlung des Gesamtstickstoffes die Analysenproben in stark schwefelsaurer Lösung bei hoher Temperatur unter Zusatz eines Katalysators aufgeschlossen und der organisch gebundene reduzierte Stickstoff in Ammonium überführt. Durch nachfolgende Wasserdampfdestillation wird das Ammonium in eine Säurevorlage überführt und sein Gehalt titrimetrisch bestimmt. Diese Methode eignet sich sowohl für partikuläre Proben als auch wässrige Lösungen. Der wasserlösliche organische Stickstoff ist einer der wichtigesten Nährstoffparameter von Böden für die Pflanzenproduktion, da er den an die Pflanzenwurzeln nachlieferbaren Stickstoff kennzeichnet und damit ein Maß für den Düngezustand des Bodens darstellt.

Die wasserlösliche organische Substanz eines Bodens wird üblicherweise als "dissolved organic carbon" oder abgekürzt DOC des wässrigen Eluates einer Bodenprobe bezeichnet. Dieser Parameter ist ein weiteres Maß für den Düngezustand des Bodens, da er Rückschlüsse auf den Humusgehalt sowie die Bodenfauna und Bodenflora ermöglicht. Für die Bestimmung des DOC eignet sich die Messung des entstandenen Kohlendioxids mittels Infrarotspektroskopie nach erfolgter Oxidation der organischen Substanz im wässrigen Eluat einer Bodenprobe. Die Anwendung dieser Methode geschieht meist automatisiert mit Hilfe geeigneter Apparate. Die Anmeldung DE 199 54 783 A1 "Verfahren zur Ermittlung des Gehaltes an Stickstoff und/oder Kohlenstoff aus der organischen Substanz in Böden, Erden, Sedimenten, und Komposten" beschreibt ein Verfahren zur Ermittlung des Gehaltes an Stickstoff und/oder Kohlenstoff aus der organischen Substanz in Böden, Erden, Sedimenten und Komposten, bei dem aus der Probe Fraktionen mittels Wasser und/oder chemischen Mitteln, die über dispergierende Eigenschaften verfügen und gleichzeitig Tauschprozesse begünstigen, aufbereitet, analysiert und verglichen werden. Dabei wird aus nasschemischen aufbereiteten Flüssigfraktionen der Stickstoff- und Kohlenstoffgehalt ermittelt und mittels Reflexionsspektroskopie im Wellenlängenbereich von 400 nm bis 2500 nm in Küvetten für Flüssigkeiten vermessen. Alternativ dazu können auch die Flüssigfraktionen mit Kieselgel wie Celite vermischt, getrocknet und in einer Pulverküvette gemessen werden. Die Messgrößen in Form der Absorptionswerte bei bestimmten Wellenlängen werden kalibriert und quantitativ nach dem Gehalt an Stickstoff und Kohlenstoff durch einen rechnerischen Vergleich der Werte aus den nasschemischen Analysen ausgewertet. Die ermittelten Beziehungsgrößen in Form von Gruppen- und Standardkalibrationen werden in der Folge für die Auswertung weiterer Probenmesswerte aus der Reflexionsspektroskopie oder Transmissionsspektroskopie im Wellenlängenbereich von 400 nm bis 2500 nm genutzt.

Auch die Anmeldung DE 100 55 821 A1 "Verfahren zur Ermittlung der Verfügbarkeit von Stickstoff und/oder Kohlenstoff in Böden und von Erden, Sedimenten und Komposten für das Pflanzenwachstum zur Quantifizierung der Stickstoffdüngung" beschreibt ein Verfahren zur Ermittlung der Verfügbarkeit von Stickstoff und/oder Kohlenstoff in Böden und von Erden, Sedimenten und Komposten für das Pflanzenwachstum zur Quantifizierung der Stickstoffdüngung.

Die Bestimmung des Ammoniums erfolgt analog zur Bestimmung des Gesamtstickstoffes ohne Aufschluss der Proben. Ammonium-Stickstoff kann auch alternativ nach erfolgter Farbreaktion photometrisch bestimmt werden. Auch dieses Verfahren ist für die Anwendung auf feste Proben und wässrige Lösungen geeignet, sofern von der festen Probe vorher eine wässrige Suspension hergestellt wurde und diese dem Verfahren unterzogen wird.

Kalium, Natrium, Calcium, Magesium werden durch die erwähnte Flüssigkeitschromatographie oder durch die ICP oder Atomabsorptionsspektroskopie analysiert.

Die Anmeldung AT 501 135 B 1 "Verfahren zum hygienischen Versand und zur Schnellanalytik von pastösen und flüssigen eutrophen Stoffen mittels Trägertechnik und spektroskopischer Methode" beschreibt ein Verfahren, bei dem flüssige eutrophe Stoffe, die sowohl Flüssigkeit als auch Feststoffpartikel beinhalten, von einem Zeolith-Trägermaterial absorbiert werden und nach Trocknung in der Pulverküvette spektroskopisch auf Gesamt-Stickstoff, Ammonium, Phosphor, Kalium, Natrium, Calcium und Magnesium untersucht und quantifiziert werden. Diese Methode weist eine mäßige Genauigkeit in der quantitativen Analyse der genannten Parameter auf, da unregelmäßig große und geformte Partikel stark zur Messungenauigkeit beitragen können.

Für die agrartechnische Beurteilung der gemessenen Nährstoffparameter ist das jeweils angewendete Zubereitungsverfahren des wässrigen Eluates der Bodenprobe von großer Bedeutung. Es existieren eine Reihe von Methoden zur Elution von Bodenproben, die sich in der Elutionsdauer, in der angewendeten Elutionstemperatur, im Zusatz von Puffersubstanzen oder säureaktiven Substanzen zum Elutionsmittel unterscheiden. Eine umfangreiche Darstellung der angewandten Methoden findet sich im VDLUFA-Methodenbuch, Band 1, des Verbandes deutscher landwirtschaftlicher Untersuchungs- und Forschungsanstalten. Die Analysenergebnisse von Böden sind oftmals unterschiedlich, je nach gewählter Methode der Eluatzubereitung.

Die angeführten Analysenverfahren nach dem Stand der Technik repräsentieren nur eine Auswahl der gegenwärtig angewandten Analyseverfahren. Alternative Verfahren, die hier nicht beschrieben sind, werden in der Praxis durchaus angewandt. Allen naßchemischen Analysenvefahren ist nachteilig gemeinsam, dass ihre Verwendung mit hohem Aufwand an Personal, Apparatetechnik und zum Teil umweltschädlichen und teuren Materialien und Chemikalien verbunden ist. Allen spektroskopischen Verfahren, die eine Analyse der Inhaltsstoffe durch Vermessung des Transmissions- oder Reflexionsspektrums im Wellenlängenbereich zwischen 400 nm und 2500 nm durchführen, ist aufgrund von Störstoffen wie Wasser oder Feststoffpartikel nachteilig gemeinsam, dass sie nur mäßig genaue Analysenwerte liefern. Lediglich das in der bereits erwähnten Anmeldung DE 199 54 783 A1 genannte Verfahren ermöglicht es, die wasserlöslichen Stickstoff- und/oder Kohlenstoffgehalte aus dem Eluat einer Bodenprobe durch Aufsaugen auf ein oberflächenaktives Trägermaterial wie vorzugsweise CELITE^{®} und nachfolgender Trocknung bei 50°C sowie nachfolgender Messung und statistischer Auswertung des Reflexionsspektrums im Wellenlängenbereich zwischen 400 nm und 2500 nm zu ermitteln. Es ist aber durch dieses Verfahren nicht möglich, zusätzlich und gleichzeitig die anderen genannten Nährstoffparameter wie Kalium, Natrium, Calcium, Magnesium oder Phospor zu bestimmen.

Ein weiterer Nachteil dieses Verfahrens ist, dass die Absorptionskraft von CELITE^{®} für Wasser in dieser Anwendung zu gering ist und der Trocknungsvorgang des Trägermaterials daher ungleichförmig verläuft, wodurch es zu ungleichförmiger Beladung des Trägermaterials mit den zu bestimmenden Substanzen kommt. Dieser Effekt ist in der Chemie als der "Chromatographie-Effekt" bekannt, der sich darin äußert, dass jene Trägermaterial-Fraktionen, die zuletzt abtrocknen, eine höhere Konzentration der gelösten Substanzen beinhalten. Dieser Effekt bewirkt im Messverfahren eine hohe Ungenauigkeit, der die Methode als wenig geeignet für die praktische Anwendung erscheinen lässt.

Aufgabe der Erfindung ist es daher, ein Verfahren zu ermitteln, bei dem das Eluat einer Bodenprobe so aufbereitet wird, dass die simultane quantitative Messung der Nährstoffparameter der wasserlöslichen organischen Substanz, des wasserlöslichen organischen Stickstoffs und Kohlenstoffs, sowie der wasserlöslichen Anteile von Kalium, Natrium, Calcium, Magnesium und Phosphor in einem einfachen und kostengünstigen Verfahren ermöglicht.

Dies wird erfindungsgemäß dadurch gelöst, dass ein Verfahren zur Bestimmung von Bodenproben zur Anwendung kommt, nach dem eine definierte Menge einer Bodenprobe in einem flüssigkeitsdichten Behältnis mit einer definierten Menge eines flüssigen Elutionsmittels eluiert wird, und danach das Eluat von einem pulverförmigen Zeolithmaterial durch ein Sieb hindurch aufgesaugt wird, und das so gewonnene eluatbeladene Zeolithmaterial getrocknet und mittels Reflexionsspektroskopie im Bereich der Wellenlängen von 400 nm bis 2500 nm analysiert wird, wobei aus dem Reflexionsspektrum die quantitativen Gehalte der wasserlöslichen organischen Substanz, des wasserlöslichen organischen Stickstoffs und Kohlenstoffs, sowie der wasserlöslichen Anteile von Kalium, Natrium, Calcium, Magnesium und Phosphor der Bodenproben durch statistischen Vergleich mit den Reflexionsspektren einer Gruppe von gleich behandelten Bodenproben ähnlicher und bekannter chemischer Zusammensetzung mittels bekannter multivariater Berechnungsmethoden ermittelt wird.

Als Elutionsmittel können alle in der Bodenanalytik verwendeten Elutionsmittel eingesetzt werden. Beispielsweise besteht die Möglichkeit, das Eluat als Heißwasserextrakt oder Kaltwasserextrakt herzustellen, und als Elutionsmittel chemische Puffersysteme zu wählen, wie sie beispielsweise von Matsumoto S. und Ae N. in "Characteristics of extractable soil organic nitrogen determined by using various chemical solutions and ist significance for nitrogen uptake by crops", Soil Science and Plant Nutrition, Band 50, Heft 1, Seiten 1-9 (2004) oder von Miyazawa K. und Murayama T. in "Heterogeneity of neutral phosphate buffer extractable soil organic matter", Soil Science and Plant Nutrition, Band 53, Seiten 1-6 (2007) beschrieben sind. Besonders gut für für die erfindungsgemäße Anwendung eignet sich ein Phosphat-Borat-Puffersystem

Zeolithe sind Alumosilikate mit großer innerer Oberfläche und hohem Absorptions- und Kationenaustauschvermögen. Eine umfassende Beschreibung von Zeolithen findet sich in der Sekundärliteratur, beispielsweise in Van Bekkum H., Flanigen E.M., Jacobs P.A., Jansen J.C. (Hrsg.): Introduction to zeolite science and practice. Verlag Elsevier, Amsterdam (2001). Das eingesetzte Zeolithmaterial hat folgende Funktionen zu erfüllen: Die Partikelgrössen des pulverförmigen Zeolithmaterials müssen größer sein als die Maschenweite des zum Einsatz gelangenden Siebes. Dies wird dadurch erreicht, dass ein Sieb mit der Maschenweite zwischen 0,45 µm und 5 µm gewählt wird, und das Zeolithmaterial Partikelgrössen zwischen 6 µm und 500 µm aufweist.

Als eingesetztes Zeolithmaterial können alle natürlichen und synthetischen Zeolithe eingesetzt werden, es eignet sich aber vorzugsweise natürliches Klinoptilolithmineral mit einem Klinoptilolithgehalt von über 50%, besonders bevorzugt ein Klinoptilolithgehalt von über 75%. Ein genaueres Messergebnis wird durch den Einsatz eines vorbehandelten Zeolithmaterials erreicht, bei dem der Großteil der natürlicherweise im Zeolith befindlichen austauschbaren Kationen durch H⁺-Ionen oder eine einzige Sorte von Kationen wie beispielsweise Natriumionen ersetzt werden, da das Zeolithmaterial dadurch die Messung der Gehalte von Kalium, Natrium, Calcium, Magnesium und Phosphor nicht stört. Die Herstellung des so vorbehandelten Zeolithmaterials kann beispielsweise durch Infiltration mit einer 0,1 ― 2,0 molaren Salzsäure oder Schwefelsäure oder Salpetersäure und nachfolgendem Waschen mit deionisiertem Wasser erfolgen, oder aber durch Infiltration mit einer 0,5 ― 5 molaren Natriumchloridlösung und nachfolgendem Waschen. Die Infiltrationslösungen der Säuren oder Salze sollen mindestens die gleiche Masse aufweisen wie der zu behandelnde Klinoptilolith, um eine weitgehende Entfernung der austauschbaren Kationen im Zeolith zu bewirken. Auch sollte die Infiltrationslösung mindestens 1 Stunde (h) lang auf den Klinoptilolith einwirken und die Einwirkungstemperatur mindestens 18°C betragen. Anstelle von Natriumchlorid können auch Natriumnitrat, Natriumsulfat oder die entsprechenden Kalisalze der Salzsäure, Schwefelsäure oder Salpetersäure verwendet werden. Sofern Lösungen von Natriumsalzen zur Vorbehandlung eingesetzt werden, ist die Analyse von Natrium aus Bodenproben nicht möglich. Auch für die Verwendung von Kalisalzen gilt, dass in diesem Fall die Analyse von Kalium nicht möglich ist.

Der Vorteil dieser Erfindung liegt darin, dass mit hoher Genauigkeit eine einfache, rasche und kostengünstige Analyse der wichtigsten wasserlöslichen Bodennährstoffe mit hoher Genauigkeit erreicht werden wird. Für die praktische Handhabung ist es sehr bedeutend, dass am Markt für Analytikzubehör bereits geeignete Siebvorrichtungen für die erfndungsgemäße Anwendung verfügbar sind. So wird beispielsweise für die Futtermittelanalyse ein durch keramische Halterungen formstabiles zylindrisches Sieb in der Bezeichnung FiberCup^{®} angeboten, das ohne weitere Veränderung als Sieb für die erfindungsgemäße Anwendung herangezogen werden kann. Allerdings sind auch andere Siebvorrichtungen, sofern sie säurestabil sind sowie die geeignete Maschenweite und ein Füllvolumen zwischen 10 ml und 50 ml aufweisen.

Ein weiterer Vorteil der Erfindung besteht darin, dass die Bodenelution und die Absorption der gelösten Stoffe auf dem Trägermaterial in einem einzigen Schritt erfolgt und die nachfolgende Trocknung im selben Siebgefäß, das bereits während der Elution eingesetzt wurde, erfolgen kann. Der erfindungsgemäße Zeolith in der vorgeschriebenen Körnung besitzt zusätzlich die nützliche Eigenschaft, dass er während des Trocknens leicht zusammenklumpt und schrumpft. Dadurch löst sich der Siebkuchen leicht vom Sieb ab und ist rasch von diesem in eine Pulverküvette zur spektroskopischen Vermessung überführbar. Die Klumpenbildung ist lediglich schwach ausgeprägt, sodass die Klumpen mit einem Spatel oder Mörser leicht und ohne große Kraftanstrengung zu zerbröseln sind, um ein gleichförmiges Pulver herzustellen.

Die Erfindung besitzt außerdem den Vorteil, dass infolge der hohen Saugkraft des Zeoliths und der Absorption der gelösten Stoffe im Innern der nanoporösen Zeolithstruktur kein Chromatographie-Effekt auftritt und eine gleichförmige Konzentrationsverteilung der zu messenden Stoffe auf und in dem Trägermaterial gegeben ist. Dies ist ein entscheidender Vorteil gegenüber anderen Trägermaterialien wie Kieselgel und Celite. Dieser Vorteil bewirkt eine höhere Messgenauigkeit gegenüber der Vermessung mit Celite.

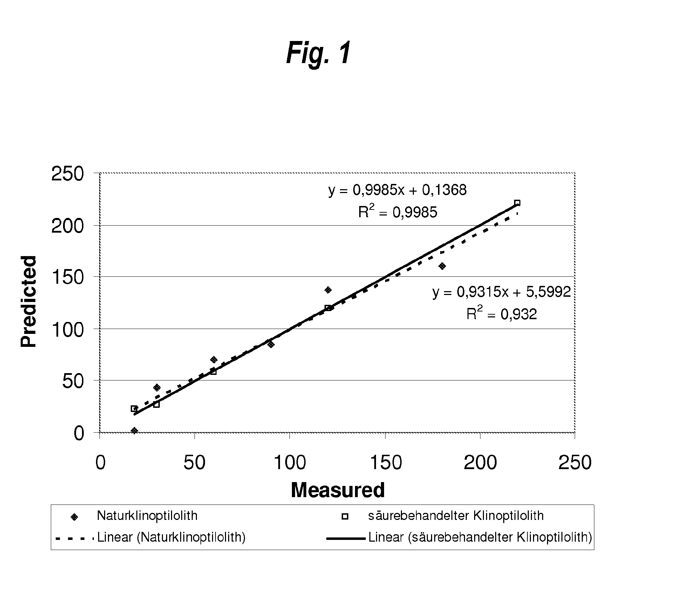

Die Erfindung wird im folgenden anhand von 1 Beispiel näher erläutert, wobei die Fig. 1 ein Liniendiagramm ergänzend zu Beispiel 1 zeigt.

Figur 1 zeigt ein Liniendiagramm mit den Messwerten der Messserie 1 und 2 des Beispiels 1. Im Diagram ist für jede Messserie eine Trendlinie eingetragen und die Geradengleichung angegeben.

### Beispiel 1:

In 2 Messreihen wurde wie folgt verfahren: Jeweils 5 Gramm (g) von insgesamt 6 Bodenproben österreichischer Braunerde wurden bei 55°C luftgetrocknet, und mit 25 ml eines Elutionsmittels, bestehend aus Phosphat-Borat-Puffer eine Stunde bei 39°C im Überkopfschüttler eluiert. Danach wurden die Proben samt Elutionsmittel jeweils in 100 ml-Bechergläser gefüllt. Für jede Probe wurde ein zylinderförmiges Sieb mit der Maschenweite von 5 µm und dem Füllvolumen von 20 ml, das kommerziell und standardisiert unter dem Namen FiberCup^{®} erhalten wurde, mit 14 g pulverförmigen Zeoliths mit einem Klinoptilolith-Gehalt von 84 % (w/w) und Partikelgrössen zwischen 6 und 300 µm befüllt und jeweils in die Bechergläser gestellt. Nach 10 min wurden jeweils etwa 7 ml Flüssigkeit vom Zeolith aufgesaugt, wie durch nachfolgende Wägung festgestellt wurde. Die Siebe wurde aus dem Becherglas genommen, an der Aussenseite mechanisch grob gesäubert und nachfolgend für 2 h bei 55°C im Trockenschrank getrocknet. Danach wurde jeweils das Zeolithmaterial aus dem Sieb genommen, in eine Pulverküvette für die Reflexionsspektroskopie gegeben und das Reflexionsspektrum durch Messung im Wellenlängenbereich zwischen 400 nm und 2500 nm ermittelt. Die erhaltenen Reflexionsspektren wurden in einer multivariaten Auswertung den durch konventionelle Analytik erhaltenen Messergebnissen von Kalium gegenüber gestellt. Der Unterschied zwischen den beiden Messserien bestand darin, dass in der Messserie 1 ein unbehandelter Naturklinoptilolith als Trägermaterial herangezogen wurde, und in der Messserie 2 ein säurebehandelter Klinoptilolith, der durch 1-stündige Behandlung mit 1 molarer Salzsäure bei 22°C und nachfolgendem Waschen erhalten wurde. Es zeigte sich, dass die Messgenauigkeit bereits beim unbehandelten Naturklinoptilolith mit einem Korrelationsfaktor R² = 0,932 bereits sehr gut lag. Die Säurebehandlung erzielte allerdings ein noch besseres Ergebnis mit einem Korrelationsfaktor von R² = 9985 und einer nur minimalen Abweichung der Korrelationskurve vom Nullpunkt.

## Patentansprüche

1. Verfahren zur Bestimmung von Bodenproben, nach dem eine definierte Menge einer Bodenprobe in einem flüssigkeitsdichten Behältnis mit einer definierten Menge eines flüssigen Elutionsmittels eluiert wird, und danach das Eluat von einem pulverförmigen Zeolithmaterial durch ein Sieb hindurch aufgesaugt wird, und das so gewonnene eluatbeladene Zeolithmaterial getrocknet und mittels Reflexionsspektroskopie im Bereich der Wellenlängen von 400 nm bis 2500 nm analysiert wird, wobei aus dem Reflexionsspektrum die quantitativen Gehalte der wasserlöslichen organischen Substanz, des wasserlöslichen organischen Stickstoffs und Kohlenstoffs, sowie der wasserlöslichen Anteile von Kalium, Natrium, Calcium, Magnesium und Phosphor der Bodenproben durch statistischen Vergleich mit den Reflexionsspektren einer Gruppe von gleich behandelten Bodenproben ähnlicher und bekannter chemischer Zusammensetzung mittels bekannter multivariater Berechnungsmethoden ermittelt wird.

2. Verfahren nach Anspruch 1, wenn als Zeolithmaterial Klinoptilolith zum Einsatz kommt.

3. Verfahren nach den Ansprüchen 1 und 2, wenn der Klinoptilolith eine Korngrößenverteilung zwischen 6 µm und 500 µm aufweist.

4. Verfahren nach den Ansprüchen 1, 2 und 3, wenn der Klinoptilolith durch die Behandlung von mindestens 1 molarer Salzsäure und / oder Schwefelsäure und / oder Salpetersäure im Gewichtsverhältnis Säurelösung/Klinoptilolith von mindestens 1 mindestens 1 h bei einer Temperatur von mindestens 18°C behandelt wurde und danach mindestens 1 mal mit deionisiertem Wasser abgespült wurde.

5. Verfahren nach Anspruch 1, wenn als Sieb ein zylinderförmiges, durch Halterungen aus keramischem Material stabilisiertes Sieb, das am unteren Zylinderboden geschlossen ist, am oberen Zylinderboden jedoch offen ist, zum Einsatz kommt.

6. Verfahren nach Anspruch 1 und 5, wenn das zylinderförmige Sieb eine Maschenweite zwischen 0,45 µm und 5 µm aufweist.
